# EUROPEAN PATENT SPECIFICATION

(11) **EP 0 988 039 B1**
(45) Date of publication and mention of the grant of the patent: **23.01.2008**
(21) Application number: 96943748.2
(22) Date of filing: 24.12.1996
(51) Int. Cl.: A61K 31/485

(54) **TOPICAL APPLICATION OF OPIOID DRUGS SUCH AS MORPHINE FOR RELIEF OF ITCHING AND SKIN DISEASE OR IRRITATION**
TOPISCHE ANWENDUNG VON OPIOIDEN, WIE MORPHIN, ZUR LINDERUNG VON JUCKREIZ UND HAUTKRANKHEITEN SOWIE HAUTREIZUNGEN
APPLICATION LOCALE DE MEDICAMENTS OPIOIDES TELS QUE LA MORPHINE POUR SOULAGER LES DEMANGEAISONS ET LES MALADIES DE PEAU OU L'IRRITATION

(43) Date of publication of application: 29.03.2000
(73) Proprietor: Elkhoury, George, F., Long Beach, CA 90815 (US)
(72) Inventor: Elkhoury, George, F., Long Beach, CA 90815 (US)
(74) Representative: Weiss, Wolfgang
(86) International application number: PCT/US1996/019924
(87) International publication number: WO 1998/027985

(56) References cited:
- EP-A- 0 398 720
- EP-A- 0 897 726
- US-A- 3 852 456
- US-A- 4 416 886
- US-A- 5 352 680
- US-A- 5 589 480

## Description

### 1. Field of the Invention

The present invention is directed to the use of opioid analgesic agents such as morphine for the manufacture of a medicament for topical application. Specifically, the present invention relates to the use of such a medicament by topical application to produce an antipruritic effect in an area of itching and/or skin disease or irritation, in the substantial absence of transdermal migration of the opioid analgesic agent into the blood stream.

### 2. Background Information

Itching (pruritus) is an irritating sensation that has some parallels to pain yet is quite different. The mechanisms of pruritus have been closely studied but still are not fully understood. The most likely initiators of the sensation of itching are chemical substances, including endogenous substances like a histamine, prostaglandin, epidermal protease, substance P, or endopeptide. Recently, it has been shown that morphine receptors may be involved in the mechanism of itching and its treatment. The use of narcotic antagonists has been proposed, such as in U.S. Patent No. 4,416,886 to BERNSTEIN et al.

The sensation of itching is conducted slowly along C nerve fibers. Initiation of the itching is understood to be the result of certain mediators acting on free nerves ending on the skin with the epidermal junction. Scratching is a unique response to the sensation of itching, thought to be a reflex to remove noxious agents from the skin surface. Scratching brings about a transient sense of relief and may even give pleasure (possibly through the stimulation of morphine receptors).

Itching may be a localized or generalized phenomenon. Localized itching can be caused by skin diseases such as psoriasis, dermatitis, eczema, scabies, or flea bites, which can all produce pruritus. Generalized pruritus can be the result of certain environmental factors, including an excessively dry or humid climate, dry centralized heating, perspiration, overbathing and harsh soap. The generalized pruritus can also be caused by primary skin disease. Generalized pruritus can also be the result of diseases such as internal disease affecting the endocrine and metabolic-hepatic-renal-hematological system, in addition to other processes such as cancer. Other causes of itching include allergic reactions to medications such as penicillin, or illicit drugs.

Treatment: The optimal therapy for generalized pruritus is treatment of the underlying disease. However, if the etiology has not been determined or if there is no specific treatment, a number of nonspecific palliative therapies exist. Treatment may be topical or systemic. The cooling effect of menthol and the anesthetic effect of phenol or camphor may offer temporary relief for some patients. Low potency topical steroids are good for short term therapy but they are not recommended for extended use.

When generalized pruritus is treated systemically, it is usually because topical measures have provided no relief. Oral antihistamines are the most commonly used. They act by competitively blocking peripheral histamine receptors, thereby blocking a mode of conduction of itch impulse. The problem with antihistamines is that they can produce a sedative effect and rather than directly controlling the itching they may be working by dulling the perception of itching. In addition, antihistamines may not be effective in all cases.

There have been multiple topical solutions to treat pruritus, including topical capsaicin, which may reduce pruritus in patients on hemodialysis. For pruritus associated with liver disease, there has been treatment with agents such as rifampin, which is mostly used for tuberculosis. However, these agents can have extreme side effects like depression of bone marrow. Of course, there are many other cases where there is no true treatment for pruritus, such as for patients who have diseases that have nonspecific therapies, for example, pruritus associated with AIDS. In those cases many kinds of treatment have been tried without any success, like ultraviolet lights, capsaicin and antihistamines, all without any major relief. Also, the ultraviolet therapy in AIDS patients is undesirable because ultraviolet light adversely affects the T4 lymphocytes and subsequently immunity.

In the treatment of acute pain, post-operative pain, or in the treatment of chronic pain (cancer pain), the use of intraspinal narcotics is increasing significantly. With intraspinal narcotics the common complication is itching, mainly in the face and in the hands. To alleviate this itching, the patients are usually treated with antihistamine and cortisone ointment. These medications, such as diphenhydramine or a topical cortisone, may not provide effective relief for the itching but in addition produce the side effects discussed above.

Regardless of its etiology, pruritus which has been present for a long period of time can cause significant frustration for both patients and physician. Thus, it is important for the physician to regularly assess the patient for possible underlying disease and attempt to find different therapies which will be helpful to the patient without causing major side effects.

Topical methods: Combating the urge of scratching is an important part of therapy. Application of a cool, moist washcloth to the area, or application of pressure with the palm of the hand near the pruritus area can abate the urge of scratching. Topical preparations may also be used for moisturizing, in addition to emollients to fight dryness, but avoiding products with sensitizing potential is vital. Bath oils are quite useful in conditions of the skin. A menthol-phenol preparation, for example Noxzema, can be helpful as a counterirritant. The major benefits of topical corticosteroids have to be weighed against all their potential side effects such as corticosteroid dependence, as well as depression of the steroid system in the body.

Oral antihistamines: Oral medications have been used to treat pruritus, and antihistamines remain the cornerstone of oral therapy. The use of traditional histamine blockers has been limited by their sedative side effects, but all these medications, in addition to working partly on the pruritus, have major side effects such as drowsiness. In addition, many of the new drugs, which have been approved by the FDA, can produce a change in heart rate.

Morphine is the prototype of the class of opioid analgesic agents which exert their effects by activating opioid receptors in or outside the central nervous system. Historically, narcotics have been used since the 18th century in the form of oral or injectable morphine or opium for pain relief. Morphine is considered to be unsurpassed as an analgesic for severe pain.

Unfortunately, morphine and other opioid analgesic agents have a number of severe side effects which hamper their widespread use and acceptance by both physicians and patients. These side effects include addiction, nausea, inhibition of breathing, somnolence and dysphoria, all of which are mediated by morphine's action within the brain. It is still the current belief that narcotics ingested or injected will enter the blood stream and travel to the brain where morphine receptors are found. In the brain, the narcotics are believed to attach to the morphine receptors and create a dullness of the pain but with all of the side effects described above. Of course, the worst potential side effect is the addiction that can occur if the morphine is used beyond a few days or weeks on a continuous basis.

Reference is made to my earlier invention, made in conjunction with Dr. Christoph Stein, Serial No. 08/291,614, filed August 17, 1994 wherein the use of a topical application of an opioid analgesic agent to alleviate pain is disclosed.

This prior application discusses in detail the use of the topical application of the opioid analgesic for the treatment of pain and this discussion is hereby incorporated by reference as though set forth in full in this application.

The European patent application EP-A-398720 discloses the use of piperazine derivatives, which are selective kappa opioid receptor agonists, for the treatment of pain.

The European patent application EP-A-897726 discloses the use of opioid K receptor agonists as antipruritic compouunds. The agonist disclosed in EP-A-897726 are described as having a higher specificity for k receptors than µ and δ receptors.

### SUMMARY OF THE INVENTION

It has now been surprisingly discovered that the topical application of opioid analgesic agents preferably in a non-transdermal carrying agent produces a therapeutic effect in an area of itching and/or skin disease or irritation. Skin diseases treatable by the present invention are those whose symptoms include itching or pruritus. The present invention offers the use of an opioid analgesic agent for the manufacture of a medicament which relieves the itching effectively and without side effects.

The present invention is based on the surprising discovery that the topical, non-systemic, and preferably, non-transdermal, application of opioid analgesic agents is effective in significantly reducing, and therefore treat pruritus in patients in need of such treatment.

Hence, the object of the present invention is the use of an opioid analgesic agent for the manufacture of a medicament for treating pruritus by topical administration, wherein said medicament comprises a therapeutically effective amount of said opioid analgesic agent, which amount is ineffective for production of systemic effects, admixed with a pharmaceutically acceptable carrying agent for topical administration, with the proviso that said agent is not an agonist having a higher specificity for K receptors than µ and δ receptors.

In particular, a further object of the present invention is the use of an opioid analgesic agent selected from the group consisting of morphine, heroin, hydromorphone, oxymorphone, codeine, hydrocodone, oxycodone, buprenorphine, methadone, meperidine, fentanyl and propoxyphene for the manufacture of a medicament for treating pruritus by topical administration, wherein said medicament comprises a therapeutically effective amount of said opioid analgesic agent, which amount is ineffective for production of systemic effects, admixed with a pharmaceutically acceptable carrying agent for topical administration.

The term opioid analgesic agents, as used herein, refers to compounds known to produce an analgesic effect through opioid receptors. When morphine is referred to individually in this application, this reference is meant to encompass other opioid analgesic agents and is not meant to mean morphine exclusively. Other ' opioid analgesic agents include, but are not limited to, compounds based on or derived from cyclazocine, piperidine, piperazine, pyrrolidine, morphiceptin, meperidine, trifluadom, benzeneacetamine, diarylacetamide, benzomorphan, alkaloids, peptides, phenantrene, and pharmaceutically acceptable salts, prodrugs, and derivatives thereof. Specific examples of compounds which are specifically contemplated by the present, invention as opioid analgesic agents suitable for use in accordance with the present invention include, but are not limited to morphine, heroin, hydromorphone, oxymorphone, codeine, hydrocodone, oxycodone, buprenorphine, methadone, meperidine, fentanyl and propoxyphene. Very preferred opioid analgesic agent compounds suitable for the use in accordance with the present invention are morphine, morphine sulfate, methadone and fentanyl. Pharmaceutically acceptable salts of the foregoing may also be employed. Suitable opioid analgesic agents, including the foregoing are also described in Goodman and Gilman's Pharmacological Basis of Therapeutics, Ninth Edition, (McGraw-Hill 1995), Chapter 23 (pp. 521-555), which is hereby incorporated by reference as though set forth in full herein. The terms opioid analgesic agent and opioid analgesic drug are used interchangably herein.

Preferably, the opioid analgesic agents of the present invention are used in an amount therapeutically equivalent to up to 5 mg of morphine per 38.7 cm² (6 square inches) of skin, preferably in an amount therapeutically equivalent to 3-5 mg per 38,7 cm² (6 square inches) of skin.

As used herein, the term non-transdermal carrying agent is defined as a vehicle into which the opioid analgesic agent is diluted, which will not significantly enhance the penetration of the opioid analgesic agent through the skin. Thus, non-transdermal carrying agents do not enhance the systemic delivery of the opioid analgesic agent. Such non-transdermal carrying agents include, but are not limited to, K-Y Gel or Jelly, Eucerine, Lanoline, Vaseline, and Aquaphor. All of these non-transdermal carrying agents are contrasted with transdermal carrying agents such as DMSO, which can significantly enhance penetration of a drug through the skin and thus the systemic delivery of the drug. A thorough description of acceptable pharmaceutical carrying agents can be found in many pharmaceutical science textbooks, for example, Remington's Pharmaceutical Sciences, Eighteenth Edition, (Mack Publishing Company 1990), the entire contents of which are incorporated by reference as though set forth herein.

According to the present invention, the carrying agent may be a liquid. Hence, pruritus may be treated in accordance with the present invention by spraying the opioid analgesic agent suspended in or admixed with a suitable non-transdermal carrying agent and sprayed (either with a manually-actuated pump or with the aid of a suitable pharmaceutically-acceptable propellant) onto the affected skin of the patient in need of treatment of pruritus. Suitable formulations for topical application of drugs by spraying the formulation containing the drug onto the skin are well known to those of ordinary skill in the art and can be routinely selected.

Consequently, the opioid analgesic agent of the invention is for administration by spraying onto the skin.

Alternatively, according to the invention the carrying agent may be a gel or a cream. In this case, the opioid analgesic agent is for administration by spreading onto the skin.

Without further elaboration, it is believed that one skilled in the art can, using the preceding description, utilize the present invention to its fullest extent. The following preferred specific embodiments are, therefore, to be construed as merely illustrative, and not limitative of the remainder of the disclosure in any way whatever.

### EXAMPLES

Initially, a topical application of opioid analgesic agents was used for five patients who were experiencing intolerable itching. The first four patients were patients who had been treated with long term intraspinal narcotics. These patients originally had significant pain of the upper extremities and lower extremities and they could not find any relief except through the injection of intraspinal narcotics. These patients were having good relief from the pain but they were unable to get over the itching. The itching in these four patients was very significant in the face and hands, so the patients discontinued the intraspinal treatment. These patients fell into very severe depression because of the constant pain. Their normal activities were suspended and they were afraid of taking the injection of intraspinal morphine because of the resultant itching which could last for as long as 24 hours. These four patients were asked to try a topical morphine gel, which was a combination of 120 cc of K-Y gel with 90 mg of morphine, and to apply 3 cc of this combination on the face and hands and any other area which was itching. After these four patients applied the topical opioid analgesic agent of the present invention, they felt relief from the itching within three or four minutes. The patients were able then to enjoy the pain relief that comes with injection of intraspinal morphine, so the patients now are being maintained, and very successfully, on the intraspinal narcotics. The typical treatment is now as follows. After the intraspinal injection, the patients take 3 or 4 cc of the topical morphine and rub it on their face, hands and arms and the itching goes away within minutes.

Another common situation relates to patients who take oral medication and develop sensitivity to it. For example, when taking antibiotics, to which the patient may be allergic, itching will occur. A fifth patient had been taking antibiotics for a medical condition, and with each administration of the antibiotics, the patient developed a rash on her legs, arms and chest with severe itching. The rash would develop as welts on the lower and upper extremities and chest, in addition to severe itching in the areas of the welts. The patient scratched all those areas without any relief. The patient was provided with the same formulation, 120 cc of K-Y gel with 90 mg of morphine, and was asked to rub that cream on her thighs, arms and chest. The patient used a total of 6 cc of this combination, which is less than 3 mg of morphine. The patient felt complete relief within minutes of rubbing the affected areas with the cream and after a period of time the skin irritation was substantially reduced.

None of the five patients reported any side effects, which is understandable because the amount of narcotics used is extremely minimal, a total of anywhere from 2 to 5 mg, depending on the area covered. Absorption from the skin is insignificant, so there are none of the side effects encountered with oral antihistamines such as diphenhydramine. There is no constipation, no nausea, no vomiting and none of the side effects encountered with the use of narcotics or steroids. The relief was uniformly high in those very severe cases which have not been helped with any other treatment. The patients were provided with a total of 120 cc, which would last them anywhere from four to five weeks. The patients were pleased with the results because they had the relief from itching without any side effects.

As to the explanation of why the topical application of narcotics relieves the itching and/or skin irritation, it has been suggested that itching is mediated by morphine receptors (for example, U.S. Patent No. 4,416,886 to BERNSTEIN et al.). All of the laboratory work previously done in other labs used a morphine antagonist for relief of itching. The present invention is the only application of a morphine agonist.

The above results were accomplished with the use of a small quantity such as less than five milligrams of the opioid analgesic agent, such as morphine, diluted to be applied to a relatively large area of skin such as 38,7 cm² (six inches square) and without any side effects such as addiction, dullness in the brain, respiratory depression, nausea, vomiting or itching. All of this was accomplished without any significant absorption of the morphine into the blood stream since the morphine was merely applied topically to the skin without any transdermal agent.

The following is a table of a representative sample of the results achieved with a number of patients listed as follows:

| PRURITUS PATIENTS | | | | |
|---|---|---|---|---|
| Patient | Disease | Treatment | Result | Previous Treatment |
| 1 | Total body itching | Morphine cream | Excellent | * Hydrocortisone |
| | | | | * Benadryl |
| | | | | |
| 2 | Face itching from epidural morphine | Morphine cream | Excellent. Medication used only twice. | * Benadryl |
| | | | | |
| 3 | Facial itching from epidural morphine | Morphine cream | Excellent Medication used only twice | * Benadryl |
| | | | Excellent Medication used only once | |
| 4 | Bee sting of hand with swelling and itching | Morphine cream | | * Atarax |
| | | | | * Benadryl |
| | | | | * Hydrocortisone |
| | | | | |
| 5 | Hand itching from psoriasis | Morphine cream | Excellent. Itching disappeared for seven days with single use. | * Hydrocortisone |
| | | | | * Benadryl |
| | | | | * Atarax |
| | | | | |
| 6 | Foot itching from | Morphine cream | Excellent Itching improved. but condition did not improve. | * Hydrocortisone |
| | Athlete Foot | | | * Atarax |
| | | | | * Mycotic agent |
| | | | | |
| 7. | Total body itching | Morphine cream | Excellent. Total relief of itching within minutes, and welt disappeared within one hour. | * Hydrocortisone |
| | | | | * Atarax |
| | | | | * Benadryl |
| | | | | |
| 8. | Facial itching from Epidural Morphine | Morphine cream | Excellent | * Benadryl |
| | | | | |
| 9 | Facial itching from Epidural Morphine | Morphine cream | Excellent | * Benadryl |
| | | | | |
| 10. | Acute Herpes Zoster with itching of chest. | Morphine cream | Excellent. Medication used every six hours for three days | * Benadryl |
| | | | | * Hydrocortisone |

| SKIN DISEASE | | | | |
|---|---|---|---|---|
| Patient | Disease | Treatment | Result | Previous Treatment |
| 11. | Acute Herpes Zoster for three weeks | Morphine Cream | Excellent Relief of pain and improved skin lesions within 3 days. | * Cortisone |
| | | | | * Zovirax |
| | | | | |
| 12. | Acute Herpes Zoster for 17 days | Morphine Cream | Excellent. Relief of pain and improved skin lesions within 2 days. | * Cortisone |
| | | | | * Zovirax |
| | | | | |
| 13. | Psoriasis of hands | Morphine Cream | Excellent Medication used once and skin improved for seven days | * Cortisone |
| | | | | |
| 14 | Psoriasis of hand | Morphine Cream | Skin lesions improved with usage. | * Cortisone |

The preceding examples can be repeated with similar success by substituting the generically and specifically described agents and/or conditions of this invention for those used in the preceding examples.

In addition to the topical application of the opioid, such as morphine, using a gel, the opioid may be applied using a variety of different topical formulations such as sprays, creams, etc. Depending upon the particular type of itching and/or skin irritation, the topical application will reduce the itching and/or skin irritation. The main advantage of the present invention is the excellent relief without the typical side effects associated with oral antihistamines or topical cortisone. The potential for the present invention is widespread and the topical application of opioid analgesic agents for itching opens up a whole new use without the prior associated problems.

Skin diseases as used herein may include psoriasis, dermatitis, eczema, or a combination thereof.

### BRIEF DESCRIPTION OF THE DRAWINGS

Figure 1 illustrates a topical application of an opioid analgesic agent using a spray; and
Figure 2 illustrates a topical application of an opioid analgesic agent using a cream or gel.

### DETAILED DESCRIPTION OF THE DRAWINGS

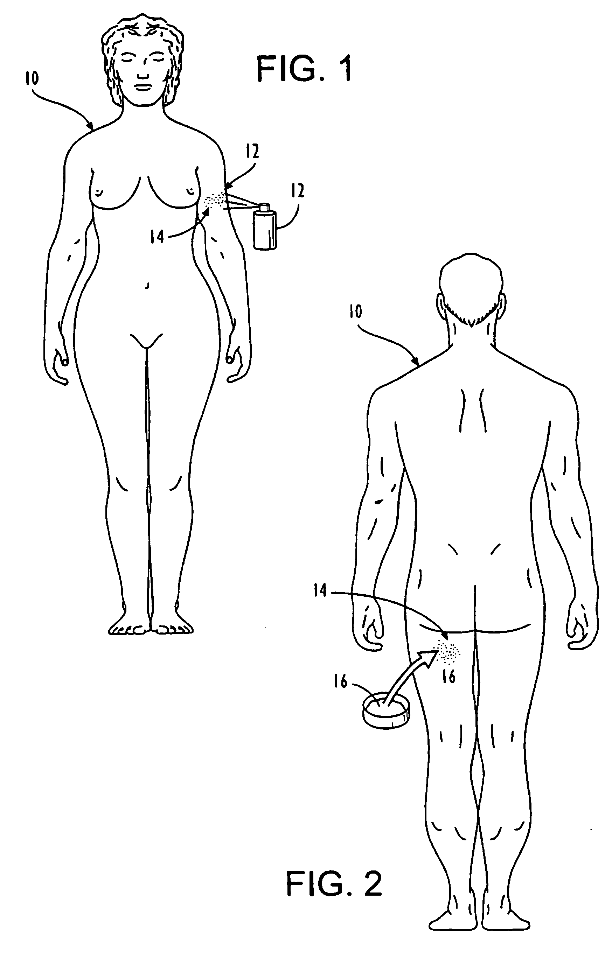

Figure 1 illustrates a method and apparatus of the present invention and specifically shows a patient 10 receiving a topical application of an opioid, such as morphine sulphate using a spray 12. In particular, a small quantity of the morphine sulphate solution is sprayed onto an area 14 of itching and/or skin irritation on a patient 10 to provide the particular relief described above. As a specific example, 90 milligrams of morphine sulphate may be diluted by 120 cc of saline to form the spray 12. The morphine sulphate is initially provided as a solution of 10 milligrams per cc so that final spray solution is 90 milligrams in a total of 129 cc. Thus, the final concentration of morphine in the spray is 0.69 milligrams per cc. The specific application may result in approximately 2 to 3 milligrams of morphine in solution to cover approximately a 232,2 cm² (6 x 6 square inch) area.

Figure 2 illustrates a patient 10 with an area 14 of itching and/or skin irritation and with an opioid, such as morphine sulphate applied topically in either a gel or a cream 16. As a specific example, 90 milligrams of morphine sulphate may be mixed with 120 cc of a topical gel such as K-Y gel. Again the morphine sulphate is initially provided in solution as 10 mg per cc and with the resultant mixture 16 being 90 mg of morphine sulphate in a total of 129 cc. The resultant gel is applied to the inflamed area 14 so that 2 to 3 mg of morphine sulphate covers an area of approximately 232,2 cm² (6 x 6 square inches). Covering a larger area either with a spray or gel will provide for the application of a greater quantity of morphine but over this larger area.

In both methods of application, as shown in Figures 1 and 2, the relief is substantial and with continued application on a periodic basis to continue this relief without any of the typical side effects provided by oral antihistamine or topical cortisone. The quantities of the applied opioid described above are illustrative only and it is to be appreciated that lesser and greater quantities may be used. However, any quantity of opioid used in the topical application of the present invention is a small fraction of the typical dosage used in other methods of opioid treatment.

It is to be appreciated that the present invention has been described primarily with reference to the use of opioid analgesic agents in the form of morphine or morphine sulphate. Other opioid analgesic agents and other forms of morphine may be used to interact with the peripheral opioid receptors which are present in peripheral areas of the body and the invention is not to be limited specifically to morphine or morphine sulphate.

From the foregoing description, one skilled in the art can easily ascertain the essential characteristics of this invention, and without departing from the spirit and scope thereof, can make various changes and modifications of the invention to adapt it to various usages and conditions.

Although the invention has been described with reference to particular embodiments, it is to be appreciated that various adaptations and modifications may be made and the invention is only to be limited by the appended claims.

## Claims

1. Use of an opioid analgesic agent for the manufacture of a medicament for treating pruritus by topical administration, wherein said medicament comprises a therapeutically effective amount of said opioid analgesic agent, which amount is ineffective for production of systemic effects, admixed with a pharmaceutically acceptable carrying agent for topical administration, with the proviso that said agent is not a K opioid receptor agonist.

2. The use of an opioid analgesic agent selected from the group consisting of morphine, heroin, hydromorphone, oxymorphone, levorphanol, codeine, hydrocodone, oxycodone, buprenorphine, butorphanol, methadone, meperidine, fentanyl, propoxyphene, pentazocine and nalbuphine for the manufacture of a medicament for treating pruritus by topical administration, wherein said medicament comprises a therapeutically effective amount of said opioid analgesic agent, which amount is ineffective for production of systemic effects, admixed with a pharmaceutically acceptable carrying agent for topical administration.

3. The use of claims 1 or 2, wherein the opioid analgesic agent is in an amount therapeutically equivalent to up to 5 mg of morphine per 6 square inches (38,7 cm²) of skin.

4. The use of claim 3, wherein the opioid analgesic agent is in an amount therapeutically equivalent to 3-5 mg of morphine per 6 square inches (38,7 cm²) of skin.

5. The use of claims 1 or 2, wherein the opioid analgesic agent is morphine.

6. The use of claim 5, wherein the opioid analgesic agent is morphine sulfate.

7. The use of claims 1 or 2, wherein the opioid analgesic agent is methadone.

8. The use of claims 1 or 2, wherein the opioid analgesic agent is fentanyl.

9. The use of claims 1 or 2, wherein the carrying agent is a liquid.

10. The use of claim 9, wherein the opioid analgesic agent is for administration by spraying onto the skin.

11. The use of claims 1 or 2, wherein the carrying agent is a gel.

12. The use of claim 11, wherein the opioid analgesic agent is for administration by spreading onto the skin.

13. The use of claims 1 or 2, wherein the carrying agent is a cream.

14. The use of claim 13, wherein the opioid analgesic agent is for administration by spreading onto the skin.

## Patentansprüche

1. Verwendung eines opioiden Analgetikums zur Herstellung eines Arzneimittels zur Behandlung von Pruritus mittels topischer Verabreichung, wobei das Arzneimittel eine therapeutisch wirksame Menge des opioiden Analgetikums umfasst, wobei die Menge zur Erzeugung von systemischen Wirkungen unwirksam ist, im Gemisch mit einem pharmazeutisch annehmbaren Träger zur topischen Verabreichung, mit der Maßgabe, dass das Mittel kein Agonist ist, der eine höhere Spezifität für κ-Rezeptoren als für µ- und δ-Rezeptoren aufweist.

2. Verwendung eines opioiden Analgetikums, welches ausgewählt ist aus der Gruppe bestehend aus Morphin, Heroin, Hydromorphon, Oxymorphon, Codein, Hydrocodon, Oxycodon, Buprenorphin, Methadon, Meperidin, Fentanyl und Propoxyphen zur Herstellung eines Arzneimittels zur Behandlung von Pruritus mittels topischer Verabreichung, wobei das Arzneimittel eine therapeutisch wirksame Menge des opioiden Analgetikums umfasst, wobei die Menge zur Erzeugung von systemischen Wirkungen unwirksam ist, im Gemisch mit einem pharmazeutisch annehmbaren Träger zur topischen Verabreichung.

3. Verwendung nach Ansprüchen 1 oder 2, wobei das opioide Analgetikum in einer Menge vorliegt, die therapeutisch bis zu 5 mg Morphin pro 6 Quadratzoll Haut entspricht.

4. Verwendung nach Anspruch 3, wobei das opioide Analgetikum in einer Menge vorliegt, die therapeutisch 3-5 mg Morphin pro 6 Quadratzoll Haut entspricht.

5. Verwendung nach Ansprüchen 1 oder 2, wobei das opioide Analgetikum Morphin ist.

6. Verwendung nach Anspruch 5, wobei das opioide Analgetikum Morphinsulfat ist.

7. Verwendung nach Ansprüchen 1 oder 2, wobei das opioide Analgetikum Methadon ist.

8. Verwendung nach Ansprüchen 1 oder 2, wobei das opioide Analgetikum Fentanyl ist.

9. Verwendung nach Ansprüchen 1 oder 2, wobei der Träger eine Flüssigkeit ist.

10. Verwendung nach Anspruch 9, wobei das opioide Analgetikum zur Verabreichung durch Sprühen auf die Haut vorgesehen ist.

11. Verwendung nach Ansprüchen 1 oder 2, wobei der Träger ein Gel ist.

12. Verwendung nach Anspruch 11, wobei das opioide Analgetikum zur Verabreichung durch Verteilen auf der Haut vorgesehen ist.

13. Verwendung nach Ansprüchen 1 oder 2, wobei der Träger eine Creme ist.

14. Verwendung nach Anspruch 13, wobei das opioide Analgetikum zur Verabreichung durch Verteilen auf der Haut vorgesehen ist.

## Revendications

1. Utilisation d'un agent analgésique opioïde pour la fabrication d'un médicament pour traiter le prurit par administration topique, où ledit médicament comprend une quantité thérapeutiquement efficace dudit agent analgésique opioïde, laquelle quantité est inefficace pour la production d'effets systémiques, en mélange avec un agent vecteur pharmaceutiquement acceptable pour l'administration topique, à condition que ledit agent ne soit pas un agoniste ayant une plus grande spécificité pour les récepteurs κ que pour les récepteurs µ et δ.

2. Utilisation d'un agent analgésique opioïde choisi dans le groupe consistant en la morphine, l'héroïne, l'hydromorphone, l'oxymorphone, la codéine, l'hydrocodone, l'oxycodone, la buprénorphine, la méthadone, la mépéridine, le fentanyle et le propoxyphène pour la fabrication d'un médicament pour traiter le prurit par administration topique, où ledit médicament comprend une quantité thérapeutiquement efficace dudit agent analgésique opioïde, laquelle quantité est inefficace pour la production d'effets systémiques, en mélange avec un agent vecteur pharmaceutiquement acceptable pour l'administration topique.

3. Utilisation selon les revendications 1 ou 2 où l'agent analgésique opioïde est en une quantité thérapeutiquement équivalente à jusqu'à 5 mg de morphine pour 6 pouces carrés de peau.

4. Utilisation selon la revendication 3 où l'agent analgésique opioïde est en une quantité thérapeutiquement équivalente à 3-5 mg de morphine pour 6 pouces carrés de peau.

5. Utilisation selon les revendications 1 ou 2 où l'agent analgésique opioïde est la morphine.

6. Utilisation selon la revendication 5 où l'agent analgésique opioïde est le sulfate de morphine.

7. Utilisation selon les revendications 1 ou 2 où l'agent analgésique opioïde est la méthadone.

8. Utilisation selon les revendications 1 ou 2 où l'agent analgésique opioïde est le fentanyle.

9. Utilisation selon les revendications 1 ou 2 où l'agent vecteur est un liquide.

10. Utilisation selon la revendication 9 où l'agent analgésique opioïde est destiné à l'administration par pulvérisation sur la peau.

11. Utilisation selon les revendications 1 ou 2 où l'agent vecteur est un gel.

12. Utilisation selon la revendication 11 où l'agent analgésique opioïde est destiné à l'administration par étalement sur la peau.

13. Utilisation selon les revendications 1 ou 2 où l'agent vecteur est une crème.

14. Utilisation selon la revendication 13 où l'agent analgésique opioïde est destiné à l'administration par étalement sur la peau.
